(19) Europäisches Patentamt
European Patent Office
Office européen des brevets

(11) Publication number: **0 646 373 A2**

(12) **EUROPEAN PATENT APPLICATION**

(21) Application number: **94113368.8**

(51) Int. Cl.[6]: **A61K 31/34**, A61K 31/365

(22) Date of filing: **26.08.94**

(30) Priority: **27.08.93 JP 212632/93**
**27.08.93 JP 212673/93**

(43) Date of publication of application:
**05.04.95 Bulletin 95/14**

(84) Designated Contracting States:
**DE FR GB**

(71) Applicant: **Nippon Paint Co., Ltd.**
**2-1-2, Oyodokita**
**Oyodo-ku**
**Osaka-shi**
**Osaka-fu (JP)**

(72) Inventor: **Yamamoto, Yoshikazu**
**24-1-402, Ikedakitamachi**
**Neyagawa-shi,**
**Osaka-fu (JP)**
Inventor: **Miura, Yasutaka**
**4-7-21, Tsukahara**
**Takatsuki-shi,**
**Osaka-fu (JP)**
Inventor: **Kinoshita, Yasuhiro**
**1-10-227, Ikuno**
**Katano-shi,**
**Osaka-fu (JP)**
Inventor: **Ohigashi, Hajime**
**19, Misasagioiwa-cho,**
**Yamashina-ku**
**Kyoto-shi,**
**Kyoto-fu (JP)**
Inventor: **Koshimizu, Koichi**
**856-10, Horenyamazoenishimachi**
**Nara-shi,**
**Nara-ken (JP)**

(74) Representative: **Hansen, Bernd, Dr.**
**Dipl.-Chem. et al**
**Hoffmann, Eitle & Partner,**
**Patentanwälte,**
**Arabellastrasse 4**
**D-81925 München (DE)**

(54) **Inhibitor of Epstein-Barr virus expression.**

(57) Disclosed is an inhibitor of Epstein-Barr virus expression comprising at least one compound of the group consisting of the formula I:

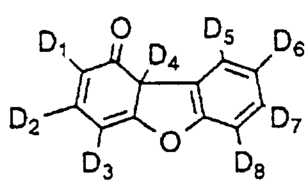

(I)

wherein each of $D_1$, $D_2$, $D_3$, $D_4$, $D_6$, $D_7$ and $D_8$ represents R-, Cl-, RO- or RCO-, wherein each R represents a hydrogen atom, an alkyl group containing 1-9 carbon atoms or an alkene group containing 1-9 carbon atoms, and the formula II:.

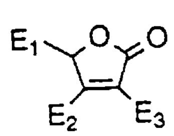

(II)

EP 0 646 373 A2

wherein each of $E_1$, $E_2$ and $E_3$ represents R-, ROOC-, RCH(OH)- or RCO-, wherein each R represents a hydrogen atom, an alkyl group containing 1-9 carbon atoms or an alkene group containing 1-9 carbon atoms. The inhibitor of the present invention is useful as antioncogenic promoter.

## FIELD OF THE INVENTION

The present invention relates to a composition containing a compound which exhibits antioncogenic promoter activity by inhibiting Epstein-Barr virus (EBV) expression in EBV latent infected human lymphoblastoid cells.

## BACKGROUND OF THE INVENTION

Recently, as a short-term detection method of an oncogenic promotor, an assay using Epstein-Barr virus expression as an index has been established by Ito et al (Cancer Letter, 13, 29-37(1981)). Using this assay system, it is possible to conduct screening for an antioncogenic promoter activity by detecting induction of EBV EA (early antigen of Epstein-Barr virus) induced by tumor promoter such as TPA (12-O-tetradecanoylphorbol-13-acetate) or teleocidin B in EBV latent infected human lymphoblastoid cells. Inhibitor of Epstein-Barr virus expression may be extremely useful as antioncogenic promoter (see Ito et al., Cancer Letter 13, 29-37 (1981)). In addition, using this assay system, inhibitory action against the expression of Epstein-Barr virus of flavonoides and triterpenes are revealed.

## OBJECT OF THE INVENTION

An object of the present invention is to provide a compound other than flavonoides or triterpenes having an activity to inhibit expression of Epstein-Barr virus in latent EBV infected cells and pharmaceutical composition containing the same.

## SUMMARY OF THE INVENTION

According to the present invention, there is provided an inhibitor of Epstein-Barr virus expression comprising at least one compound of the group consisting of the formula I:

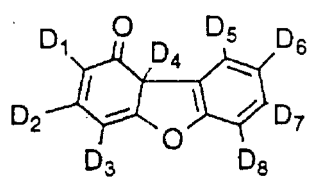

(I)

wherein each of $D_1$, $D_2$, $D_3$, $D_4$, $D_5$, $D_6$, $D_7$ and $D_8$ represents R-, Cl-, RO- or RCO-, wherein each R represents a hydrogen atom, an alkyl group containing 1-9 carbon atoms or an alkene group containing 1-9 carbon atoms and the formula II:

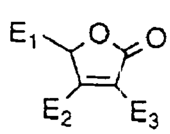

(II)

wherein each of $E_1$, $E_2$ and $E_3$ represents R-, ROOC-, RCH(OH)- or RCO-, wherein each R represents the same as above.

## DETAILED EXPLANATION OF THE INVENTION

In the above formula I, it is preferable that each of $D_6$ and $D_7$ represents $R_1$- or $R_1$CO-, wherein each $R_1$ represents hydrogen atom or $-CH_3$. In addition, it is also preferable that each of $D_5$ and $D_7$ represents $R_1$O-. Further, each of $D_1$-$D_6$ preferably represents -OH, $-CH_3$ or $-OCH_3$.

In the above formula II, it is preferable that each of $E_1$ and $E_3$ represents $R_1$- or $R_1$CO-, wherein each $R_1$ represents the same as above. In addition, $E_2$ preferably represents $R_1$OOC-. Further, each of $E_1$-$E_3$ preferably represents $-CH_3$, -COOH or $-C_{13}H_{27}$.

The compounds of the present invention may be obtained by chemical synthesis or isolated from extraction of natural lichens or lichen cultures.

A method for synthesis of the compounds of the present invention has been described in, for example, Prog. Chem. Organ. Nat. Prod. 45 103-234(1984). On the other hand, in order to isolate the compounds of the present invention from lichens or lichen cultures, a usual extraction method for natural material may be used. For example, the lichens or lichen culture are dipped in an organic solvent such as acetone, then, by means of chromatography such as liquid chromatography the desired compound may be isolated from the crude extract. The term "lichen culture" used herein means a culture of lichen cells, algae cells or indifferent symbiotic cultured tissue wherein both cells are present.

Examples of lichens are *Teloschistaceae, Physciaceae, Buelliaceae, Usneaceae, Anziaceae, Parmeliaceae, Candelariaceae, Lecanoraceae, Pertusariaceae, Acarosporaceae, Umbilicariaceae, Cladoniaceae, Baeomycetaceae, Streocaulaceae, Lecideaceae, Gyalectaceae, Asterothyriaceae, Stictaceae, Peltigeraqceae, Pannariaceae, Coccocaripiaceae, Placynthiaceae, Heppiaceae, Collemataceae, Lichnaceae, Graphidaceae, Thelotremataceae, Diploschistaceae, Verrucariaceae, Pyrenulaceae, Strigulaceae, Sphaerophoraceae, Caliciaceae, Cypheliaceae, Lecanactidaceae, Opegraphaceae, Arthopyreniaceae, Arthoniaceae, Dictyonemataceae, Clavariaceae, Agariaceae* and the like.

The compound of the present invention may be orally administrated as it is, or administrated in a form of an oral pharmaceutical composition combined with at least one pharmaceutically acceptable adjuvant. It may also be a formation absorbed percutaneously, like a cosmetic composition. As the dosage form of the oral or percutaneous composition, for example, there are solid preparations such as tablet, granulate and capsule, and liquid preparations.

The solid preparation may contain conventional excipient, such as silicic anhydride, synthesized aluminum silicate, lactose, cornstarch and crystalline cellulose, binders, such as carboxymethylcellulose and polyvinyl pyrrolidone, corrigent, sweeteners, colorants and the like.

The liquid preparation may be an aqueous or oily suspension, solution, syrup and the like, or it may be formed into a dried article which can be redissolved with a suitable vehicle before use. The liquid preparation may contain normal emulsifiers, such as lecithin and sorbitan monooleate, auxiliary emulsifiers, such as sorbit syrup, methylcellulose and gelatin, non-aqueous vehicles, such as coconut oil and peanut oil, antioxidants, colorants, flavors and the like.

The dosage of the compound of the present invention varies depending on a factor such as constitution of the patient, conditions to be treated and the like. Generally, when it is administered to an adult patient, the dosage may be 0.01 to 0.1 g, preferably 0.08 to 0.1 g a day.

The inhibitor of Epstein-Barr virus expression of the present invention is extremely useful as an antioncogenic promotor.

The present invention will be further explained in detail in the following examples, which, however, are not to be construed as limiting the scope to the examples.

EXAMPLE

Following two inhibitors of the present invention were estimated in the EBV EA induction test. One was pure usnic acid of the formula:

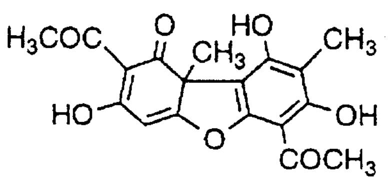

which was extracted and isolated from *Alectoria ochloreuca*. The another was pure lichesterinic acid of the formula:

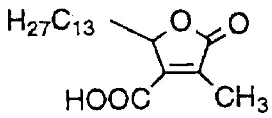

which was extracted and isolated from *Cetraria islandicia*. 3-oxo ursolic acid was used as a positive control for inhibition of Epstein-Barr virus expression. The inhibitors were dissolved in dimethyl sulfoxide respectively to prepare sample solution.

EBV EA induction test using human lymphoid non-producer Raji cells were conducted by a modified method of Ito et. al (Cancer letter 13, 29-37(1981)). EBV of Ito et. al (Cancer letter 13, 29-37(1981)). EBV latent infected human lymphoid Raji cells were cultured in RPMI 1640 medium containing 4% serum and antibiotic. Raji cells (5 x $10^5$ cells/ml) were cultured in the basic medium containing Lactic acid (3 mM), Teleocidin B (20 ng/ml) with or without one of the inhibitors (5 x $10^{-5}$ M). After cultivation at 37 °C for 48 hours, smears were made from the cell suspension and the EBV EA expressing cells were stained using a conventional indirect immunofluorescence technique and high titer EBV-positive sera obtained from nasopharyngeal carcinoma patients. In each assay, at least 500 cells were counted and the ratio of EBV EA-positive cells were recorded. The activities of the inhibitors were described by inhibition % of the ratio of EBV-EA positive cells compared with the ratio obtained by the group without inhibitor. The results were shown in Table 1.

Table 1

| composition | inhibition % |
|---|---|
| 3-oxo ursolic acid | 81 |
| Usnic acid | >99 |
| lichesterinic acid | >99 |

## Claims

1. An inhibitor of Epstein-Barr virus expression comprising at least one compound selected from the group consisting of compounds of the formula I:

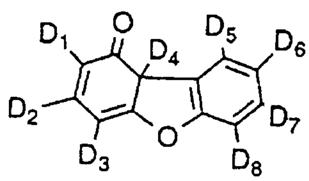 (I)

wherein each of $D_1$, $D_2$, $D_3$, $D_4$, $D_5$, $D_6$, $D_7$ and $D_8$ represents R-, Cl-, RO- or RCO-, wherein each R represents a hydrogen atom, an alkyl group containing 1-9 carbon atoms or an alkene group containing 1-9 carbon atoms, and the formula II:

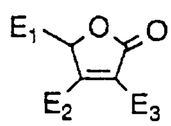 (II)

wherein each of $E_1$, $E_2$ and $E_3$ represents R-, ROOC-, RCH(OH)- or RCO-, wherein each R represents the same as above.

**2.** An inhibitor according to claim 1 wherein each of $D_6$ and $D_7$ represents $R_1CO$-, wherein each $R_1$ represents hydrogen atom or $-CH_3$.

**3.** An inhibitor according to claim 1, wherein each of $D_5$ and $D_7$ represents $R_1O$-, wherein each $R_1$ represents hydrogen atom or $-CH_3$.

**4.** An inhibitor according to claim 1, wherein each of $E_1$ and $E_3$ represents $R_1$- or $R_1CO$-, wherein each $R_1$ represents hydrogen atom or $-CH_3$.

**5.** An inhibitor according to claim 1, wherein $E_2$ represents $R_1OOC$-, wherein each $R_1$ represents hydrogen atom or $-CH_3$.

**6.** Pharmaceutical composition comprising an inhibitor of Epstein-Barr virus expression according to claim 1 combined with at least one pharmaceutically acceptable adjuvant.

**7.** Use of an inhibitor of Epstein-Barr virus expression according to claim 1 in the manufacture of a medicament for the treatment of a disease wherein an antioncogenic promotor is indicated.